# EUROPEAN PATENT APPLICATION

(11) **EP 1 967 078 A1**
(43) Date of publication of application: **10.09.2008**
(21) Application number: 07004765.9
(22) Date of filing: 08.03.2007
(51) Int. Cl.: A23L 1/212, A61K 8/97, A61K 36/185

(54) **Process and apparatus for preparing pomegranate extracts**

(71) Applicant: Probelte Pharma, S.A., 30100 Espinardo Murcia (ES)
(72) Inventor: López Más, José A., 30840 Alhama de Murcia (ES); Streitenberger, Sergio, A., 30120 El Palmar Murcia (ES); Peñalver Mellado, Marcos, 30007 Murcia Murcia (ES); Martinez Ortiz, Pedro, 30150 La Alberca Murcia (ES)
(74) Representative: Gislon, Gabriele

(57) **Abstract**

Ellagitannins are extracted from whole pomegranate fruit, by carrying out extraction of said product at a temperature within the range of 4°C to 30°C and at a pH within the range of 3.5 to 5.0, with a following purification step on adsorption resins and elution with basic solution.
A claim is directed to an ellagitannin containing product having a punicalagin content of at least 0.5%.

## Description

Process and apparatus for preparing pomegranate extracts.

The present invention relates to a process and an apparatus for preparing pomegranate extracts. More particularly, the invention relates to the production of ellagitannins-containing products to be used as a source of ellagitannins from the pomegranate in food, medical and cosmetic industries. Ellagitannins are a family of phenolic compounds including punicalagins and derivatives of ellagic acid.

Recent studies had shown that pomegranate juice and pomegranate extracts, contain a number of bioactive compounds, particularly polyphenols; among these polyphenols, ellagitannins are of outstanding biological significance in view of its antioxidant, antimicrobial and radical scavenging activity.

Studies have shown that pomegranate juice has the higher antioxidant properties than any other drink, such as blueberry juice, cranberry juice, green tea or red wine. Punicalagins α and β anomers are predominant ellagitannins in pomegranate juice, and the major responsible of antioxidant properties of the juice. Nevertheless, research pointed out that combination of punicalagins α and β with other ellagitannins such as ellagic acid and its glycosidated derivatives and other minor pomegranate ellagitannins produces an important synergistic effect enhancing healthy benefits of the juice.

Punicalagins α and β anomers are mainly present in pomegranate husk, and they pass to the primary juice during pressing for juice extraction. Punicalagins α and β have the following formula: 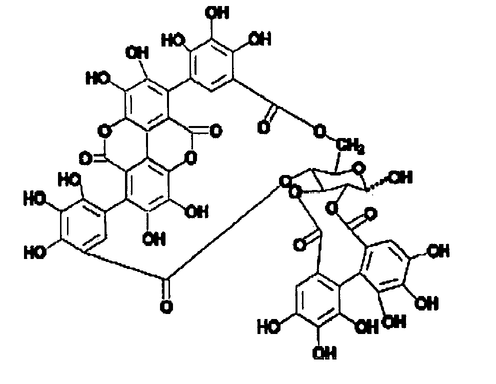 Production of extracts containing phytochemicals (e.g. ellagitannins) from pomegranate was and is actively investigated; an efficient extraction process could be very profitable.

A recent profusion of pomegranate extracts, standardized to 40 % ellagic acid, has appeared in the marketplace. Numerous studies conclude that synergy among the various pomegranate phytochemicals and not simply the concentration of ellagic acid, is a key factor for assessing the healthy benefits of pomegranate extracts,. Ellagic acid concentration in final products is likely to have an optimum therapeutic range, which very likely is less than 40 %. Moreover, ellagic acid overall effectiveness depends on the bioavailability of the compounds in which it is introduced in the body, the detection of ellagic acid in human plasma being probably dependent on the consumption of larger ellagitannins such as punicalagins, because when free ellagic acid is administrated orally, it is poorly absorbed. Recent research lines encouraged to designing and engineering pomegranate extracts to maximize therapeutic and chemopreventive synergy have emerged. US 2006/0211635 discloses purification of pomegranate ellagitannins from fruit husk, a by-product of the commercial juice industry. The process includes the steps of preparing, by a variety of methods known in the art ( e.g. blending) an aqueous suspension from pomegranate husk containing the ellagitannins from the pomegranate, removing the suspended solids to yield an aqueous solution, removing the ellagitannins onto a resin surface (e.g. XAD-16 resin) from the aqueous solution, eluting the resin surface with a solvent, preferably lower alkanols as ethanol or methanol, and separating the ellagitannins from the eluant (e.g. rotaevaporation under vacuum of solvent). This process has the drawbacks of requiring the use of organic solvents, even toxic ones such as methanol, and of generating an extract than doesn't contain several phytochemicals (anthocyanins, etc) exclusively present in the pomegranate juice fraction, when in fact the juice is the product consumed by the people since ancestral times, to which recent studies attribute the healthy benefits obtainable from pomegranate.

W02006/127832 discloses a method to produce a pomegranate extract from by-products of the juice industry. Solids from pomegranate, pericarp, inner membrane and seeds are mixed with an aqueous solution and heated optimally to 43-71 °C adding enzymes that partially degrade the pomegranate solids. After that, the process continues as follows: remaining insoluble solids are removed, by a variety of methods known in the art (e.g. microfiltration) to produce the extract containing pomegranate phytochemicals, and the resulting liquid extract is concentrated in a evaporator under vacuum, pasteurized, re-concentrated in the evaporator, cooled, blended and standardized. The above described process includes several thermical treatments where the extract is subjected to heating (2 h at 43-71 °C and pasteurization). It is known than pomegranate juice pasteurization produces free ellagic acid trough degradation of larger ellagitannins, such as punicalagins, diminishing the health benefits of drinking the juice. These steps generate several by-products that are difficult to remove and, in addition, the use of pomegranate solids coming from the juice industry generates an extract than doesn't contain some phytochemicals (minor ellagitannins, anthocyanins, etc) exclusively present in the pomegranate juice fraction. For this reason W02006/127832 discloses at one of the embodiments the combination of pomegranate extract and juice, that in fact provides a broad spectrum of the phytochemicals present in the whole pomegranate, but has the disadvantage of the high caloric value due to the sugars present in both the juice and the extract.

Summarizing, the above mentioned techniques have the disadvantage that they are either using only by-products of the juice industry, or applying harsh conditions (methanol for purification, thermal treatments).

It is an aim of the present invention to solve the above mentioned problems and to provide a process of producing pomegranate extract that is simple, effective, not expensive, that can provide water soluble punicalagins rather than solvent soluble ellagic acid, and that provides the broad spectrum of the different natural organic chemicals which are present in the whole pomegranate fruit.

Such aim is achieved by means of the present invention that provides a process according to claim 1. This process provides for the blending in water of the entire, i.e. the whole, pomegranate fruits, previously washed, with an ordinary mixer at room temperature. In the blending step the mixer blades chop up the fruit pieces to obtain a suspension/dispersion of the fruit particles in water. The pH of the homogenised mixture that results from the blending is within the range of 3.5 to 5.0. The extract is clarified by physical methods known in the art, e.g. by filtering and/or centrifuging, to remove the insoluble solids from the aqueous solution, and to obtain a clarified solution substantially free of insoluble solids.

According to a preferred embodiment of the invention the above steps are followed by the steps of loading the product thus obtained in a chromatographic column of a resin selected from adsorbent non-ionic resins and of eluting the ellagitannins retained in said chromatographic column by shifting the pH with a basic solution, preferably a basic buffer solution, most preferably obtained with sodium bicarbonate buffer. The elution is completed by a step of displacing with demineralised water.

According to a further aspect of the invention, the liquid product is concentrated by nanofiltration and/or reverse osmosis concentration. According to a further step, after chromatographic purification and nanofiltration and/or reverse osmosis concentration, the resulting liquid product is brought to a solid form, e.g. by freeze-drying, vacuum rotaevaporation or spray drying, with or without carriers such as maltodextrines.

A further object of the invention are the ellagitannins containing products as obtainable according to the above mentioned process. These products may be in solid form and are characterized by having a punicalagins content of at least 0.5% (w/w) and a purity of at least 55% and preferably of at least 70% and more preferably of at least 80%.

A further object of the invention is an apparatus for carrying out the process as above discussed, characterized according to claim 12.

The invention provides several advantages over the prior art techniques.

First of all, the extraction step is carried out, according to the present invention, using whole pomegranate fruits and water, keeping the natural pH of the extract and operating at room temperature, the resulting extraction of water-soluble ellagitannins is almost completed in about half an our, without significant formation of by-products. The extraction step according to the present invention allows to obtain, in a short time, a very good yield in water-soluble ellagitannins.

The extraction step, carried out according to the invention, provides for another advantage. The exclusive use of water avoids the extraction of huge amounts of the slightly water-soluble ellagic acid. In fact, the extraction step results in a composition of the pomegranate extract completely soluble in water.

In addition, the extraction step is preferably followed by at least a purification step, which is carried out by loading the product obtained from the extraction step, previously clarified to remove remaining insoluble solids, in at least one chromatographic column and eluting the ellagitannins retained in said chromatographic column shifting the pH with sodium bicarbonate buffer solution and displacing with demineralised water. This means that no organic solvents are contacted with the ellagitannins extract, thus obtaining a pomegranate extract that is particularly suitable to be used in the alimentary, cosmetic and pharmaceutical field.

Another advantage of the present invention is due to the purification step, that allows to separate the sugar fraction from the ellagitannins fraction. This purification step provides a "sugar-free" pomegranate extract, with all of the benefits of the pomegranate but without the calories, characterized by a high content on punicalagins and relatively little ellagic acid, which can be directly concentrated and dried without the drawbacks associated with high sugar content extracts (e.g. sticky materials). Always according to the present invention, the possibility to obtain a solid final product that is not mixed with any carrier, for example maltodextrines, gives the opportunity to formulate pomegranate extract according to its final intended use and according to any formal requirement, possibly required.

The invention will now be further disclosed in greater detail with reference to the enclosed non-limiting drawings wherein:
- Fig.1 is a schematic view of an apparatus according to the invention; and
- Fig. 2A is a HPLC chromatogram of a pomegranate extract obtained according to one embodiment of the invention process.
- Fig. 2B is a HPLC chromatogram of pure punicalagins anomers isolated from the pomegranate extract.
- Fig 2C is a HPLC chromatogram of commercially available ellagic acid.

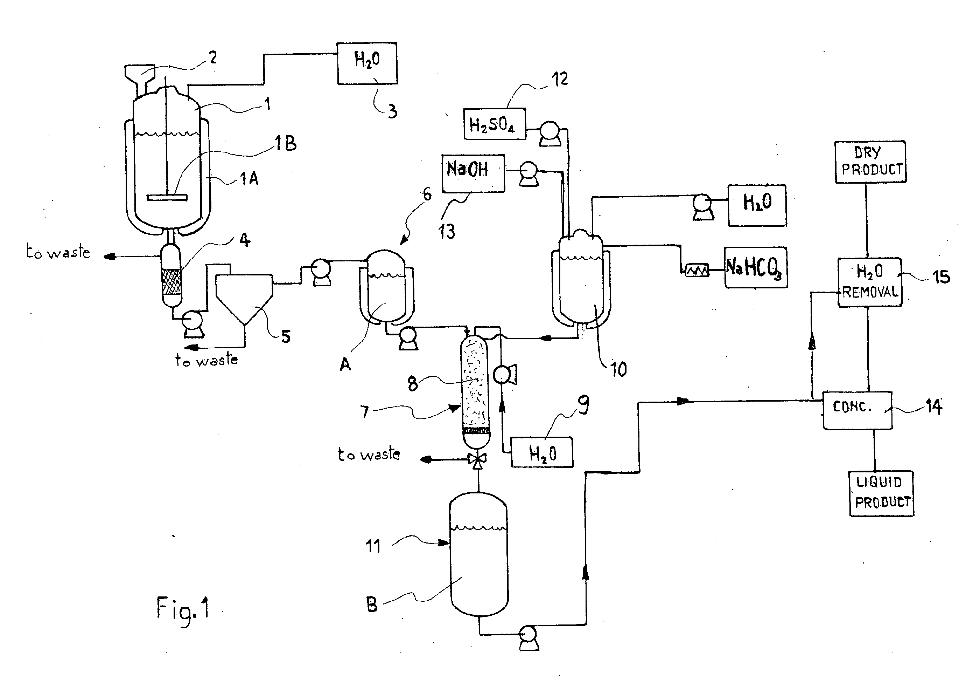

With reference to fig. 1, the invention apparatus comprises a blending means, or mill, 1, in which the extraction according to the invention process is carried out.

The mill 1 is provided with feeding means 2 for feeding to it the starting materials, namely the whole pomegranate fruits, i.e. the entire fruit comprising skin, husk, pulp, seeds etc, preferably previously cut in two halves or cut into pieces, for an easier operation of the blades 1B of the mill/blender 1.

Mill 1 is also provided with a supply of demineralised water 3; usually, the ratio water to solids is within the range of 1:1 to 1:2 v/w, i.e. between 1.0 and 0.5 liters of water per 1.0 Kg of wet pomegranate. As already mentioned, the extraction is carried out on the entire fruit, including seeds and pulp, and the juice of the fruit is therefore dissolved in the dispersion, or suspension, of fruit particles obtained by the mill.

The extraction process is carried out at a pH of 3.5 to 5.0, that is generally obtained without any addition of acids, this being the natural pH of the blended fruits in water. The blending step is carried out at a temperature within the range of 4 to 30°C, preferably at room temperature within the range of 10 to 20°C; mill 1 is provided with a jacket 1A for temperature control, when necessary.

Suitable blenders, or mills, are e.g. those providing 12,000 rpm of blades 1B; the blender motor is cooled to avoid overheating, if necessary. The stirring and disintegrating step of the blender is carried out until a substantially uniform suspension is obtained. In a plant, the stirring time is generally within 2.0 to 2.5 hours for an amount of 60 to 70 kg pomegranate to be treated.

The outlet of mill 1 is connected with a filter 4 for removing solids from the mixture containing ellagitannins and other phenols. The filtered portion is then sent to a further separation means, preferably a centrifuge 5, where further insoluble solids are removed from the reaction mixture to obtain a liquid substantially free from solids and suitable for the following purification and concentration steps. The clarified liquid A, after centrifugation, has a brownish colour and is preferably stored in reservoir 6 which is connected to purification means.

Purification means comprises at least one chromatographic column 7 of a resin selected from adsorbent non-ionic resins.

Suitable adsorption resins are based on non-ionic, hydrophobic, macroreticular cross-linked aromatic polymer. Such resins are typically aromatic polymers, such as styrene and divinylbenzene copolymers, which may be cross-linked. Such resins are known and are generally prepared by polymerization of the appropriate monomers. The adsorption resins used as a chromatographic resin for the purification of ellagitannins of the present invention are not particularly limited so long as they can be eluted using a slightly basic aqueous buffer solution. Examples of preferred adsorption resins include: Amberlite.RTM. XAD-4, XAD-7, XAD-1180, XAD-16 and XAD-1600 (available from Rohm & Haas); XUS-40323.00, XUS-40285.00 and XUS-40283.00 (available from Dow Chemical Co.); and SP-700, SP-825, SP850, Diaion HP 10, HP 20, HP 30, HP 40 and HP 50 (available from Mitsubishi Chemical).

These type of resins are particularly suitable for the invention process in view of their very high adsorption capacity for ellagitannins, including punicalagins and of the fact that the adsorbed punicalagins can be recovered by elution with water and little quantity of sodium bicarbonate, only, without any polar solvent such as methanol or ethanol, as was instead required by the prior art techniques using different resins. Adsorbed punicalagins are recovered substantially quantitatively. In the shown preferred embodiment, the process of the invention provides for an one-step purification on chromatographic column.

Liquid A, as obtained from the initial steps a) and b), i.e. extraction and solid separation, is charged into column 7, containing the adsorption resin 8 as above detailed. The permeate is sent to waste treatment (not shown). Demineralized water from water supply 9 is then fed to column 7 to wash the remaining unbound products. Then, a elution basic solution is loaded to the column 7. This solution preferably is a buffer solution at a pH within the range of 7.8 to 8.8, preferably of 8.0 to 8.5. Suitable buffers are Na bicarbonate, Na acetate and ammonium acetate. The preferred buffer solution is consisting on 0.5 to 4.0 bed volumes of sodium bicarbonate, prepared into elution tank 10, at a concentration within the range of 500 mM to 5 mM. It is loaded into column 7, promoting the elution, by shifting, of the pH the punicalagins and other ellagitannins adsorbed onto resin 8. Again, demineralized water from water supply 9 is then fed to column 7 to complete the elution displacing the remaining unbound products and the eluted liquid is collected in reservoir 11. In the most preferred embodiment the elution buffer consist on 2 bed volumes of 50 mM sodium bicarbonate at room temperature.

The thus obtained liquid product (liquid B) has a purity in punicalagins of at least 55%, and generally of at least 70%, the purity being determined as the % of the peak areas in a chromatogram by HPLC at 360 nm. The recovery of retained punicalagins from the resin is at least 85% and is generally at least 90%.

Fig. 1 also shows a source of sulphuric acid 12 that is connected to column 7 for surface activation of resin 8 and a source of NaOH 13 or other suitable base for regeneration of the same. Liquid B can be concentrated in concentration means 14, e.g. by evaporation or TFF, preferably by nanofiltration or reverse osmosis, to a punicalagin content that can reach 10%.

In a further step of the invention process, the liquid products obtained by the previously discussed steps are dried in dryer means 15, e.g. freeze-dryer, vacuum rotoevaporator or preferably by spray-dryer, to produce a solid final product. This drying step is preferably carried out on liquid product B, after concentrating as above disclosed. The drying step can in one embodiment make use of carriers suitable for the final use of the dry product; suitable carriers are e.g. maltodextrines, lactose, caseinates etcetera.

Suitable drying techniques are known in the art and comprise spray drying (in a prefereably embodiment without the use of carriers), freeze-drying and water evaporation under vacuum. The resulting products will have a punicalagins content of 0.5% to 5.0% (w/w) if a carrier is used, the punicalagins content can reach up to about 65% (w/w) if no carrier is used. It should be noticed that the invention process provides a purified liquid product B that is sugar-free: this results in the great advantage that liquid B can be concentrated and evaporated to a dry powder without carriers.

The invention will now be further disclosed with reference to the following non-limiting examples.

### EXAMPLE 1

### Pomegranate ellagitannins extraction from whole fruit, clarification of the aqueous phase

1000 g of a sample of pomegranate fruits are mixed with 1000 ml of demineralized water. The obtained mixture is blended for a few minutes. After that, the aqueous phase is separated from the solid residue, by filtering on a filter. The solid phase, retained on the filter, is washed with 100 ml of demineralized water, and the water coming from this washing operation is collected with the aqueous phase previously recovered. The aqueous phase, approximately 1700 ml, is then centrifuge refined to eliminate solid particles passed through the filter. After solid elimination, 1495 ml of crude aqueous extract, containing 9.62 g of punicalagin α, β anomers, with a HPLC purity of 68.8%, are obtained.

### EXAMPLE 2

### Adsorption punicalagins purification

A sample of 1495 ml of crude aqueous extract containing 9.62 g of punicalagin α, β anomers obtained according to Example 1, is loaded on a column containing a adsorption resin. In this example, XAD 1180 was used but other adsorption resins may be used. The liquid phase recovered at the end of the column, does not contain any punicalagins. After permeate is discarded, resin is washed with 1 bed volume of demineralised water to remove the remaining unbound products. Punicalagins are continuously eluted using a pH shift elution strategy. pH shift is promoted passing through the resin 2 bed volumes of 50 mM sodium bicarbonate. Next, we complete the elution passing demineralised water until at least 90% of the initially loaded punicalagins are recovered. The eluted phase contains approximately 9.42 g of punicalagins with an HPLC purity of about 73.4%.

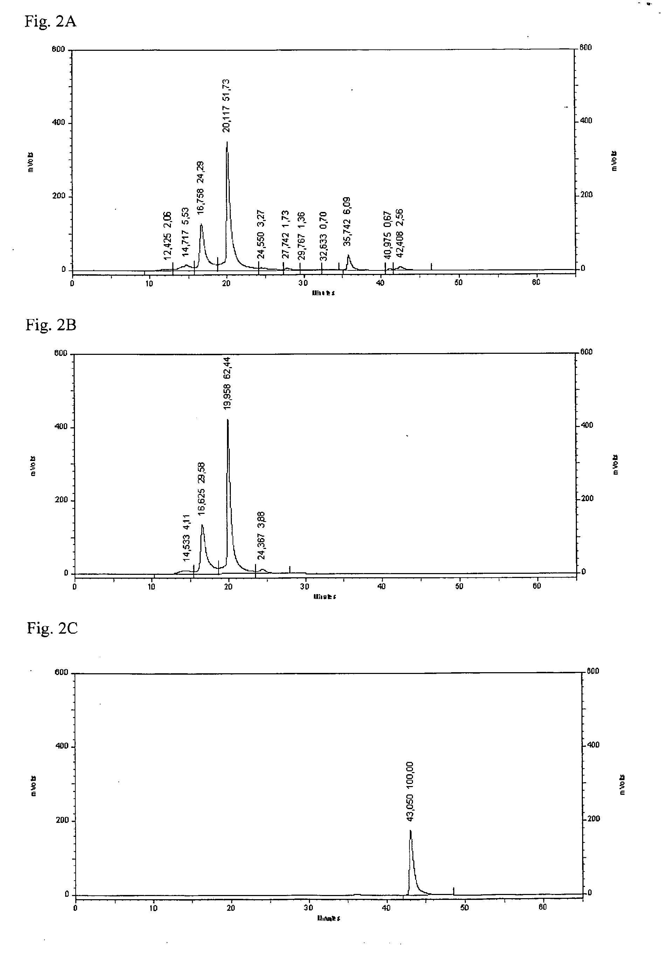

Fig.2A shows the HPLC chromatogram of the obtained purified product. In this figure it can be seen that besides the two peaks at 16 and 20 min, corresponding to the punicalagins, other products are present. Namely, punicalins, ellagic acid glycoside, free ellagic acid, etc.

Figure 2B is the HPLC of puniclagins obtained from the invention process, further purified to provide a standard.

### EXAMPLE 3

### Concentration of pomegranate extract enriched in punicalagins by nanofiltration

A sample of 60 I of crude aqueous extract containing 348 g of punicalagins obtained in a pilot plant according to Example 2, is concentrated using a nanofiltration pilot plant equipped with a polymeric membrane, in order to obtain a pomegranate extract concentrate containing 5.8% of punicalagins.

### EXAMPLE 4

### Spray-drying of the purified aqueous extract enriched in punicalagins

A sample of 5.82 I of purified aqueous extract containing 338 g of punicalagins obtained according to Example 3, is slowly feed into a spray-drier. A peristaltic pump is used to feed the spray-dryer, which is previously equilibrated with an inlet air temperature of 135°C. The feeding speed is adjusted in order to obtain an outlet air temperature which is less than 90°C. 595.6 g of an yellowish brown powder, with a moisture of 4.5% (Karl Fischer), an ellagic content of 0.4 %, and a punicalagins richness of 56.6%, are obtained.

## Claims

1. A process of producing a pomegranate extract from a suspension of blended fruit in water, **characterized in** comprising the step of:
a) carrying out said extraction of the entire blended fruit in water at a temperature within the range of 4°C to 30°C and at a pH within the range of 3.5 to 5.0.

2. A process according to claim 1, wherein said extraction is carried out in a mill and the extraction temperature is within the range of 8°C to 25°C.

3. A process according to claim 1 or 2, wherein the duration of said extraction step is within the range of 15 to 150 minutes.

4. A process according to any claim 1 to 3, further comprising the step of:
b) subsequently treating the extraction mixture of step a) to remove solids and obtain a clarified aqueous solution (A).

5. A process according to any previous claim, further comprising the steps of:
c) loading the product obtained from step b) in a chromatographic column of a adsorbent non-ionic resin;
d) eluting the products retained in said chromatographic column by shifting the pH with a basic solution, and displacing the products with demineralised water.

6. A process according to claim 5, wherein said shifting of the pH is carried out with a sodium bicarbonate elution buffer solution.

7. A process according to any claim 4 to 6, further comprising the step of:
e) concentrating the liquid product eluted from the resin by nanofiltration.

8. A process according to any previous claim, further comprising the step of:
f) water removal until the product is in a solid form.

9. An ellagitannins containing product as obtainable through a process according to any of the previous claims, and having a punicalagins content of at least 0.5% (w/w) with a punicalagins purity of at least 55% (by HPLC 360nm).

10. An ellagitannins containing solid product according to claim 9, having a punicalagins content of at least 0.5% (w/w) with a punicalagins purity of at least 55% (by HPLC 360nm), an ellagic content of no more than 3%, and a total phenol content of at least 35%.

11. A ellagitannins containing solid product according to claim 9, having a punicalagins content of at least 50% (w/w) with a purity of at least 80% (by HPLC 360nm), an ellagic content of no more than 0.5 %, and a total phenols content of at least 40%.

12. An apparatus for producing a pomegranate extract, comprising means of extraction of said ellagitannins from the fruit, **characterized in** comprising pomegranate blending means for carrying out said extraction from an aqueous suspension of the entire fruit particles at a temperature within the range of 4°C to 30°C and at a pH within the range of 3.5 to 5.0; and means for treating the extraction mixture to obtain a clarified aqueous solution.

13. An apparatus according to claim 12, further comprising at least one chromatographic column of a resin selected from an adsorbent non-ionic resin that is a macroreticular cross-linked aromatic polymer.

14. An apparatus according to any claim 12 to 13, further comprising nanofiltration means.

15. An apparatus according to any claim 12 to 14, further comprising water removal means.

16. An apparatus according to claim 15, wherein said means for water removal comprise a spray-dryer system.
